(19) Europäisches Patentamt
European Patent Office
Office européen des brevets

(11) Veröffentlichungsnummer: **0 271 733 A2**

(12) **EUROPÄISCHE PATENTANMELDUNG**

(21) Anmeldenummer: **87117032.0**

(22) Anmeldetag: **19.11.87**

(51) Int. Cl.4: **C07D 495/04** , A61K 31/415 , //(C07D495/04,333:00,235:00)

(30) Priorität: **22.11.86 DE 3639926**

(43) Veröffentlichungstag der Anmeldung: **22.06.88 Patentblatt 88/25**

(84) Benannte Vertragsstaaten: **AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Lang, Hans-Jochen, Dr.**
**Rüdesheimer Strasse 7**
**D-6238 Hofheim am Taunus(DE)**
Erfinder: **Rösner, Manfred, Dr.**
**Unter den Buchen 7**
**D-6239 Eppstein/Taunus(DE)**
Erfinder: **Weidmann, Klaus, Dr.**
**Talweg 11**
**D-6242 Kronberg/Taunus(DE)**
Erfinder: **Rippel, Robert, Dr.**
**Frankfurter Strasse 66**
**D-6238 Hofheim am Taunus(DE)**
Erfinder: **Herling, Andreas W., Dr.**
**Dieburger Strasse 43**
**D-6072 Dreieich(DE)**

(54) **Substituierte Thienoimidazoltoluidin-Derivate, Verfahren zu ihrer Herstellung, sie enthaltende pharmazeutische Zubereitungen und ihre Verwendung als Magensäuresekretionshemmer.**

(57) Thienoimidazoltoluidine der Formel I

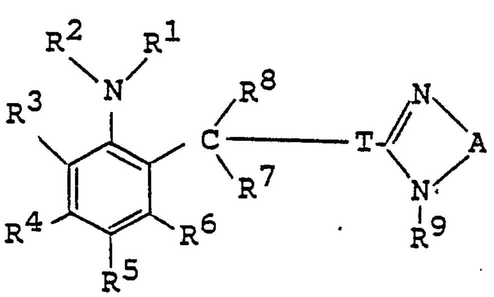

(I)

worin
A für

EP 0 271 733 A2

a) 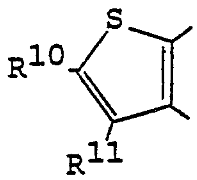 , b) 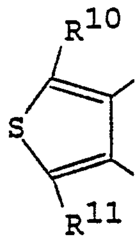 , c) 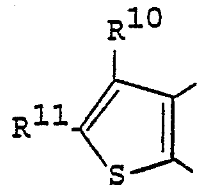

steht, T -S-, -SO-oder $-SO_2$-bedeutet und $R^1$ bis $R^8$ die in der Beschreibung angegebenen Bedeutungen haben sowie ein Verfahren zu ihrer Herstellung, sie enthaltende pharmazeutische Zubereitungen und ihre Verwendung als Magensäuresekretionshemmer werden beschrieben.

## Substituierte Thienoimidazoltoluidin-Derivate, Verfahren zu ihrer Herstellung, sie enthaltende pharmazeutische Zubereitungen und ihre Verwendung als Magensäuresekretionshemmer

Benzimidazol-Derivate mit magensäuresekretionshemmender Wirkung sind z.B. aus DE-A 25 48 340, EP-A 5129, DE-A 32 40 248, DE-A 33 33 314 und DE-A 35 09 333 bekannt.

Es wurde nun überraschenderweise gefunden, daß bestimmte substituierte Thienoimidazoltoluidine hochwirksame Magensäuresekretionshemmer sind.

Die vorliegende Erfindung betrifft Thienoimidazoltoluidine der Formel I

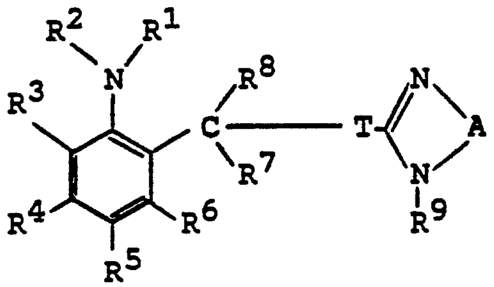

(I)

in welcher
A für

a) 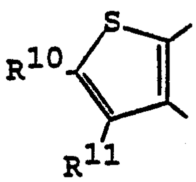 , b) 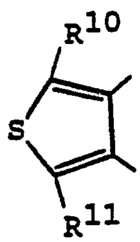 oder c) 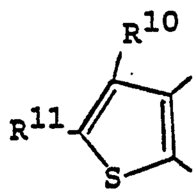

steht,

T -S-, -SO-oder $-SO_2$-bedeutet,

$R^1$ und $R^2$ gleich oder verschieden sind und Wasserstoff, ($C_1$-$C_6$)-Alkyl, ($C_3$-$C_6$)-Cycloalkyl, ($C_3$-$C_6$)-Alkenyl oder ($C_3$-$C_6$)-Alkinyl bedeuten, oder

$R^1$ und $R^2$ gemeinsam für eine Methylenkette $-[CH_2]_n$-stehen, die eine Doppelbindung enthalten kann, worin n = 2,3,4,5 oder 6 ist und worin eine Methylengruppe durch Sauerstoff, Schwefel oder $NR^{12}$ ersetzt sein kann,

$R^3$, $R^4$, $R^5$ und $R^6$ gleich oder verschieden sind und für Wasserstoff, Halogen, Cyano, Trifluormethyl, Benzyloxy, ($C_1$-$C_6$)-Alkyl-Y oder Phenyl-Y, worin Y Sauerstoff, Schwefel, Sulfinyl, Sulfonyl oder $-[CH_2]_m$-mit m = 0, 1 oder 2 bedeutet, $-CO-R^{13}$, $-SO_2NR^{14}R^{15}$, $-O-COR^{14}$, $-NR^{14}-COR^{15}$, $-NR^{14}-SO_2R^{15}$ oder $-NR^{14}R^{15}$ stehen,

$R^7$ und $R^8$ gleich oder verschieden sind und Wasserstoff oder Methyl bedeuten,

$R^9$ Wasserstoff, ($C_1$-$C_6$)-Alkanoyl, ($C_1$-$C_6$)-Alkylcarbamoyl oder eine andere physiologisch verträgliche, säurelabile und/oder unter physiologischen Bedingungen abspaltbare $N^{im}$-Schutzgruppe bedeutet,

$R^{10}$ und $R^{11}$ gleich oder verschieden sind und Wasserstoff, Halogen, Cyano, Nitro, Trifluormethyl, ($C_1$-$C_6$)-Alkyl, ($C_1$-$C_6$)-Alkoxy, ($C_1$-$C_6$)-Alkylmercapto, ($C_1$-$C_6$)-Alkylcarbonyl, ($C_1$-$C_6$)-Alkoxycarbonyl, ($C_1$-$C_6$)-Alkylcarbonyloxy, ($C_3$-$C_8$)-Cycloalkyl, Phenyl, Benzyl, Benzyloxy, Phenoxy, Phenylmercapto, Phenylsulfinyl, Phenylsulfonyl, Sulfamoyl, N-($C_1$-$C_4$)-Alkylsulfamoyl oder N,N-Di-($C_1$-$C_4$)-alkylsulfamoyl bedeuten, oder, falls A wie oben unter (a) oder (c) definiert ist, auch gemeinsam $-[CH_2]_n$-mit n = 3, 4, 5 oder 6 bedeuten können, wobei eine oder zwei nicht benachbarte $CH_2$-Gruppen gegebenenfalls durch Sauerstoff ersetzt sind,

$R^{12}$ Wasserstoff, ($C_1$-$C_4$)-Alkyl oder Acyl bedeutet,

$R^{13}$ ($C_1$-$C_5$)-Alkyl, ($C_5$ oder $C_6$)-Cycloalkyl, Hydroxy, ($C_1$-$C_4$)-Alkoxy oder $-NR^{14}R^{15}$ bedeuten,

$R^{14}$ und $R^{15}$ gleich oder verschieden sind und Wasserstoff, ($C_1$-$C_4$)-Alkyl oder Phenyl, das mit ($C_1$-$C_3$)-Alkyl, ($C_1$-$C_4$)-Alkoxy, Trifluormethyl und/oder Halogen, mono-, di-oder trisubstituiert sein kann, bedeuten, oder

$R^{14}$ und $R^{15}$ gemeinsam für eine Methylenkette $-[CH_2]_q$-stehen,

worin q = 3, 4, 5 oder 6 ist und eine Methylengruppe durch Sauerstoff ersetzt sein kann, sowie deren physiologisch verträglichen Salze.

1H-Thieno[3,4-d]imidazoltoluidin-Derivate der Formel I, worin A wie oben unter (b) definiert ist, sind bevorzugt.

T ist vorzugsweise eine -SO-Gruppe.

Weiterhin sind solche Verbindungen bevorzugt, in denen

$R^1$ und $R^2$ gleich oder verschieden sind und Wasserstoff oder $(C_1-C_4)$-Alkyl bedeuten oder gemeinsam für eine Methylenkette $-[CH_2]_n-$mit n = 4 oder 5 stehen worin eine Methylengruppe durch Sauerstoff ersetzt sein kann,

$R^3$, $R^4$, $R^5$ und $R^6$ gleich oder verschieden sind und Wasserstoff, $(C_1-C_4)$-Alkyl oder $(C_1C_4)$-Alkoxy bedeuten,

$R^7$ und $R^8$ für Wasserstoff stehen und

$R^{10}$ und $R^{11}$ gleich oder verschieden sind und Wasserstoff, Methyl oder Methoxy bedeuten.

Hervorgehoben seien Verbindungen der Formel I, worin

$R^9$ Wasserstoff oder eine physiologisch verträgliche, säurelabile und/oder unter physiologischen Bedingungen abspaltbare $N^{im}$-Schutzgruppe, insbesondere $(C_1-C_6)$-Alkanoyloxy, bedeutet.

Besonders bevorzugt sind 1H-Thieno[3,4-d]imidazoltoluidin-Derivate der Formel I worin

A vorzugsweise wie unter (b) definiert ist,

T vorzugsweise eine -SO-Gruppe bedeutet,

$R^1$ und $R^2$ gleich oder verschieden sind und Methyl oder Ethyl bedeuten,

$R^3$, $R^4$, $R^5$ und $R^6$ für Wasserstoff oder 1 oder 2 dieser Reste für $(C_1-C_4)$-Alkyl, insbesondere Methyl steht/stehen,

$R^7$ und $R^8$ jeweils für Wasserstoff stehen,

$R^9$ Wasserstoff oder $(C_1-C_6)$-Alkanoyloxy bedeutet und

$R^{10}$ und $R^{11}$ gleich oder verschieden sind und Methyl oder Methoxy bedeuten,

insbesondere

2-(2-Dimethylaminobenzylsulfinyl)-1H-thieno-[3,4-d]imidazol und

2-(2-Diethylaminobenzylsulfinyl)-1H-thieno[3,4-d]imidazol.

Halogen steht hierbei für Fluor, Chlor, Brom oder Jod.

Unter einer säurelabilen und/oder einer unter physiologischen Bedingungen abspaltbaren $N^{im}$-Schutzgruppe versteht man beispielsweise, die schon erwähnten Alkanoyl-und Alkylcarbamoyl-Reste sowie Acetyl, Trifluoracetyl, Trimethylsilylethoxycarbonyl, $(C_1-C_6)$-Alkanoyloxy, Vinyloxycarbonyl, sauer abspaltbare Gruppen vom Urethantyp wie Boc, Bpoc, Moc und Pyoc (vgl. z.B. Kontakte Merck 3/79, Seiten 14 und 16-19; Kontakte Merck 1/80, Seite 31; Schröder, Lübke, The Peptides, Vol. I New York, London 1965, Seiten 3-50).

Gegebenenfalls vorhandene chirale C-und S-Atome können sowohl in der R-als auch in der S-Konfiguration vorkommen. In solchen Fällen liegen Verbindungen der Formel I als Stereoisomerengemisch (wie Enantiomerengemisch und Diastereomerengemisch) vor.

Als Salze kommen insbesondere Alkali-und Erdalkalisalze in Frage.

Die Erfindung betrifft weiterhin ein Verfahren zur Herstellung von Verbindungen der Formel I, das dadurch gekennzeichnet ist, daß man Verbindungen der Formel II

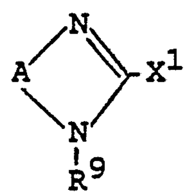

(II)

in welcher

A für

a) 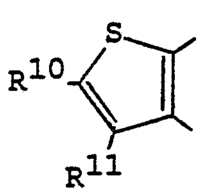 , b) 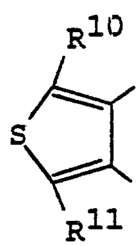 oder c) 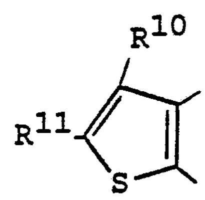

steht,
und
$R^9$ Wasserstoff, ($C_1$-$C_6$)-Alkanoyl, ($C_1$-$C_6$)-Alkylcarbamoyl oder eine andere physiologisch verträgliche, säurelabile und/oder unter physiologischen Bedingungen abspaltbare $N^{im}$-Schutzgruppe bedeutet,
$X^1$ i. eine Abgangsgruppe oder
ii. -SH oder -S bedeutet
umsetzt mit einer Verbindung der Formel III

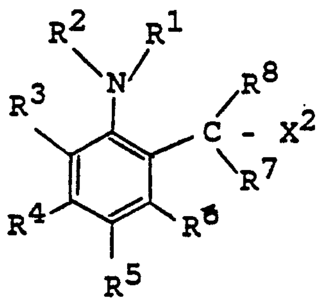

(III)

in welcher
$R^1$ und $R^2$ gleich oder verschieden sind und Wasserstoff, ($C_1$-$C_6$)-Alkyl, ($C_3$-$C_6$)-Cycloalkyl, ($C_3$-$C_6$)-Alkenyl oder ($C_3$-$C_6$)-Alkinyl bedeuten, oder
$R^1$ und $R^2$ gemeinsam für eine Methylenkette $-[CH_2]_n$-stehen, die eine Doppelbindung enthalten kann, worin n = 2,3,4,5 oder 6 ist und worin eine Methylengruppe durch Sauerstoff, Schwefel oder $NR^{12}$ ersetzt sein kann,
$R^3$, $R^4$, $R^5$ und $R^6$ gleich oder verschieden sind und für Wasserstoff, Halogen, Cyano, Trifluormethyl, Benzyloxy, ($C_1$-$C_6$)-Alkyl-Y oder Phenyl-Y , worin Y Sauerstoff, Schwefel, Sulfinyl, Sulfonyl oder $-[CH_2]_m$-mit m = 0, 1 oder 2 bedeutet, $-CO-R^{13}$, $-SO_2NR^{14}R^{15}$, $-O-COR^{14}$, $-NR^{14}-COR^{15}$, $-NR^{14}-SO_2R^{15}$ oder $-NR^{14}R^{15}$ stehen,
$R^7$ und $R^8$ gleich oder verschieden sind und Wasserstoff oder Methyl bedeuten
und
$X^2$ im obengenannten Fall i. -SH oder -S und im obengenannten Fall ii. eine Abgangsgruppe bedeutet,
sowie

i. in Verbindungen der Formel I (eine) gegebenenfalls vorhandene -S-Gruppe gewünschtenfalls zu der -SO-Gruppe oxidiert,

ii. in Verbindungen der Formel I (eine) gegebenenfalls vorhandene -SO-Gruppe gewünschtenfalls zur -S-Gruppe reduziert,

iii. Verbindungen der Formel I, worin $R^9$ für Wasserstoff steht, gewünschtenfalls acyliert,

iv. Verbindungen der Formel I, worin $R^9$ nicht Wasserstoff bedeutet, gewünschtenfalls verseift und

v. Verbindungen der Formel I gewünschtenfalls in ihre physiologisch verträglichen Salze überführt, wobei zwei oder mehr Maßnahmen i.-iv. auch in einer anderen als der angegebenen Reihenfolge ausgeführt werden können.

Setzt man gemäß der hier angegebenen Verfahrensvariante (a) Verbindungen der Formel II mit Verbindungen der Formel III um, so steht $X^1$ oder $X^2$ für eine Abgangsgruppe, die nucleophil ablösbar ist, wie Cl, Br, J, $-O-SO_2-CH_3$, $-O-SO_2-CF_3$ oder $-O-SO_2-(C_6H_4-pCH_3)$.

Die Umsetzung einer Verbindung der Formel II mit einer Verbindung der Formel III oder deren Salzen erfolgt in einem inerten Lösungsmittel wie z.B. Wasser, Methylenchlorid, Methanol, Ethanol, Aceton, Essigsäureethylester, Toluol, Tetrahydrofuran, Acetonitril, Dimethylformamid, Dimethylsulfoxid oder Gemischen dieser Lösungsmittel zweckmäßigerweise in Gegenwart einer anorganischen oder organischen Base, wie z.B. Natrium-oder Kaliumhydroxid, -carbonat, -alkoxid, -hydrid, -amid, Ammoniak, Triethylamin, Tributylamin, Pyridin bei -20 °C bis +150°C, vorzugsweise bei 0 - 80°C.

Die Verbindungen der Formel II können in Analogie zu bekannten Verfahren hergestellt werden, z.B. durch Ringschluß entsprechend substituierter o-Diaminothiophene mit Schwefelkohlenstoff (z.B. DE-A-31 32 167).

Die hierfür benötigten 2,3-, 3,4-oder 4,5-Diaminothiophene sind entweder literaturbekannt oder können in Analogie zu bekannten Verfahren hergestellt werden. Sie werden z.B. durch Reduktion entsprechend substituierter Aminonitrothiophene erhalten.

Die so erhaltenen Verbindungen der Formel I können, falls $R^9$ Wasserstoff bedeutet, in physiologisch

verträgliche Salze umgewandelt werden.

Verbindungen der Formel I mit T = -S-können ferner mit geeigneten Oxidationsmitteln in solche mit T = -SO-oder -$SO_2$-umgewandelt werden.

Diese Reaktion erfolgt in einem geeigneten, inerten Lösungsmittel wie z.B. Methylenchlorid, Chloroform, Tetrachlorkohlenstoff, 1,2-Dichlorethan, Toluol, Essigsäureethylester, Essigsäure, Trifluoressigsäure, Wasser, Methanol, Ethanol oder Gemischen derselben bei -20°C bis +150°C vorzugsweise bei -10°C bis +40°C.

Als Oxidationsmittel kommen z.B. in Betracht: Wasserstoffperoxid, Persäuren und Perester wie Peressigsäure, Trifluorperessigsäure, Monoperphthalsäure, m-Chlorperbenzoesäure und deren Ester, Ozon, Distickstofftetroxid, Jodosobenzol, N-Chlorsuccinimid, 1-Chlorbenzotriazol, Natriumhypochlorit, Kaliumperoxodisulfat, t-Butylhypochlorit, Tetrabutylammoniumperjodat oder -permanganat, 2-Arylsulfonyl-3-aryloxaziridine, Natrium-meta-perjodat, Selen-oder Mangandioxid, Cerammonnitrat, Chromsäure, Chlor, Brom, Diazabicyclo[2.2.2]octan-Bromkomplex, Dioxandibromid, Pyridiniumbromidperbromid, Sulfurylchlorid, Titantetraisopropylat/tert. Butylhydroperoxid (gegebenenfalls mit Zusatz von Dialkylestern der (R)-und (L)-Weinsäure sowie gegebenenfalls unter Zusatz einer definierten Menge Wasser).

Ebenso können isolierte, ggf. immobilisierte oxidierende Enzyme oder Mikroorganismen als Oxidationsmittel Anwendung finden.

Die Oxidationsmittel werden in äquimolaren Mengen, ggf. auch in einem geringen Überschuß von 5 - 10 Mol-% bei der Oxidation zu T = -SO-oder auch in größerem Überschuß und/oder bei höherer Reaktionstemperatur eingesetzt wenn eine Oxidation zu T = -$SO_2$-gewünscht wird.

Als erfindungsgemäße Verbindungen seien genannt, ohne die Erfindung auf diese zu beschränken:

$R^7, R^8, R^9 = H$ A = 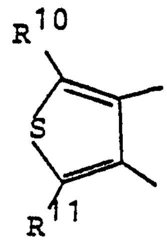 , 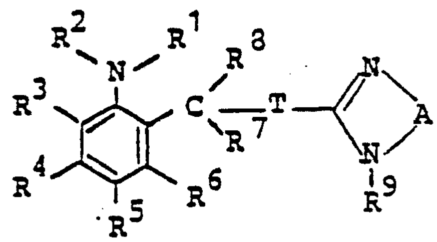

T = S

| $R^1$ | $R^2$ | $R^3$ | $R^4$ | $R^5$ | $R^6$ | $R^{10}$ | $R^{11}$ |
|---|---|---|---|---|---|---|---|
| H | H | H | Me | H | H | H | H |
| Me | H | H | Me | H | H | H | H |
| Me | H | H | Me | H | H | OMe | H |
| Me | H | H | Me | H | H | OEt | H |
| Me | H | H | Me | H | H | OMe | OMe |
| Me | H | Me | H | H | H | H | H |
| Me | H | Me | H | H | H | OMe | H |
| Me | H | Me | H | H | H | OEt | H |
| Me | H | H | H | Me | H | H | H |
| Me | H | H | H | Me | H | OMe | H |
| Me | H | H | H | Me | H | OEt | H |
| Me | H | H | H | Me | H | OMe | OMe |
| H | H | H | H | H | Me | H | H |
| Me | H | H | H | H | Me | H | H |
| Me | H | H | H | H | Me | OMe | H |
| H | H | Et | H | H | H | H | H |
| Me | H | Et | H | H | H | H | H |
| Me | H | H | Et | H | H | H | H |
| Me | H | H | H | Et | H | H | H |
| H | H | Cl | H | H | H | H | H |
| Me | H | Cl | H | H | H | H | H |
| Me | H | Cl | H | H | H | OMe | H |
| Me | H | Cl | H | H | H | OEt | H |

$R^7, R^8, R^9$ = H

T = S

A = 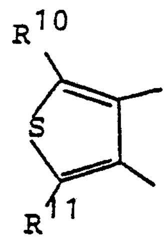

| $R^1$ | $R^2$ | $R^3$ | $R^4$ | $R^5$ | $R^6$ | $R^{10}$ | $R^{11}$ |
|---|---|---|---|---|---|---|---|
| H | H | H | Cl | H | H | H | H |
| Me | H | H | Cl | H | H | H | H |
| Me | H | H | Cl | H | H | OEt | H |
| H | H | H | H | Cl | H | H | H |
| Me | H | H | H | Cl | H | H | H |
| Me | H | H | H | Cl | H | OMe | H |
| H | H | H | H | H | Cl | H | H |
| Me | H | H | H | H | Cl | H | H |
| H | H | Br | H | H | H | H | H |
| Me | H | Br | H | H | H | H | H |
| Me | H | Br | H | H | H | OEt | H |
| Me | H | H | Br | H | H | H | H |
| Me | H | H | H | Br | H | H | H |
| Me | H | H | H | Br | H | OEt | H |
| H | H | OMe | H | H | H | H | H |
| Me | H | OMe | H | H | H | H | H |
| Me | H | OMe | H | H | H | OEt | H |
| H | H | H | OMe | H | H | H | H |
| Me | H | H | OMe | H | H | H | H |
| H | H | H | H | OMe | H | H | H |
| Me | H | H | H | OMe | H | H | H |
| Me | H | H | H | OMe | H | OEt | H |
| Me | H | H | H | H | OMe | H | H |
| H | H | OEt | H | H | H | H | H |
| Me | H | OEt | H | H | H | H | H |
| Me | H | OEt | H | H | H | OEt | H |
| Me | H | H | OEt | H | H | H | H |
| Me | H | H | H | OEt | H | H | H |
| H | H | H | H | OEt | H | H | H |
| Me | H | H | H | OEt | H | OEt | H |

$R^7, R^8, R^9$ = H

T = S

A = 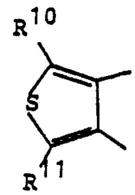

| $R^1$ | $R^2$ | $R^3$ | $R^4$ | $R^5$ | $R^6$ | $R^{10}$ | $R^{11}$ |
|---|---|---|---|---|---|---|---|
| Me | H | F | H | H | H | H | H |
| Me | H | H | F | H | H | H | H |
| Me | H | H | H | F | H | H | H |
| Me | H | H | H | H | F | H | H |
| H | H | Me | H | Me | H | H | H |
| Me | H | Me | H | Me | H | H | H |
| Me | H | Me | H | Me | H | OEt | H |
| Me | H | Me | Me | H | H | H | H |
| Me | H | Me | H | H | Me | H | H |
| Me | H | H | Me | Me | H | H | H |
| H | H | H | H | Me | Me | H | H |
| Me | H | H | H | Me | Me | H | H |
| H | H | Me | H | Cl | H | H | H |
| Me | H | Me | H | Cl | H | H | H |
| Me | H | Me | H | Cl | H | OEt | H |
| H | H | Cl | Cl | H | H | H | H |
| Me | H | Cl | Cl | H | H | H | H |
| H | H | Cl | H | Cl | H | H | H |
| Me | H | Cl | H | Cl | H | H | H |
| Me | H | Cl | H | Cl | H | OEt | H |
| Me | H | Cl | H | H | Cl | H | H |
| H | H | H | Cl | Cl | H | H | H |
| Me | H | H | Cl | Cl | H | H | H |
| Me | H | H | H | Cl | Cl | H | H |
| H | H | H | H | Cl | Cl | H | H |
| H | H | Br | H | Br | H | H | H |
| Me | H | Br | H | Br | H | H | H |
| H | H | Cl | H | OMe | H | H | H |
| Me | H | Cl | H | OMe | H | H | H |
| Me | H | Cl | H | OMe | H | OEt | H |
| H | H | OMe | H | Cl | H | H | H |
| Me | H | OMe | H | Cl | H | H | H |
| H | H | H | Cl | OMe | H | H | H |
| Me | H | H | Cl | OMe | H | H | H |
| H | H | H | H | OMe | Cl | H | H |
| Me | H | H | H | OMe | Cl | H | H |

$R^7, R^8, R^9$ = H
T = S

A = 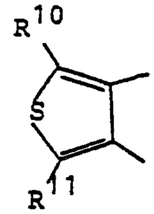

| $R^1$ | $R^2$ | $R^3$ | $R^4$ | $R^5$ | $R^6$ | $R^{10}$ | $R^{11}$ |
|---|---|---|---|---|---|---|---|
| Me | H | H | H | OMe | Cl | OEt | H |
| H | H | Cl | H | OEt | H | H | H |
| Me | H | Cl | H | OEt | H | H | H |
| H | H | Br | H | OMe | H | H | H |
| Me | H | Br | H | OMe | H | H | H |
| H | H | Me | H | OMe | H | H | H |
| Me | H | Me | H | OMe | H | H | H |
| H | H | H | Me | OMe | H | H | H |
| Me | H | H | Me | OMe | H | H | H |
| H | H | H | H | OMe | Me | H | H |
| Me | H | H | H | OMe | Me | H | H |
| Me | H | H | H | OMe | Me | OEt | H |
| Me | H | H | H | OMe | Me | OMe | OMe |
| Me | H | H | H | OMe | Cl | OMe | OMe |
| Me | H | Cl | H | OEt | H | OMe | OMe |
| Me | H | Br | H | OMe | H | OMe | OMe |
| Me | H | Me | H | OMe | H | OMe | OMe |
| Me | H | Me | H | H | H | OMe | OMe |
| Me | H | H | Me | H | H | OMe | OMe |
| H | H | H | H | Me | | OMe | OMe |
| Me | H | H | H | Me | H | OMe | OMe |
| Me | H | Et | H | H | H | OMe | OMe |
| Me | H | H | Et | H | H | OMe | OMe |
| Me | H | H | H | Et | H | OMe | OMe |
| Me | H | H | H | H | Me | OMe | OMe |
| H | H | Cl | H | H | H | OMe | OMe |
| Me | H | Cl | H | H | H | OMe | OMe |
| Me | H | H | Cl | H | H | OMe | OMe |
| H | H | H | H | Cl | H | OMe | OMe |
| Me | H | H | H | Cl | H | OMe | OMe |
| Me | H | H | H | H | Cl | OMe | OMe |
| Me | H | Br | H | H | H | OMe | OMe |
| Me | H | H | Br | H | H | OMe | OMe |
| Me | H | H | H | Br | H | OMe | OMe |
| Me | H | OMe | H | H | H | OMe | OMe |

$R^7$,$R^8$,$R^9$ = H

T = S

A = 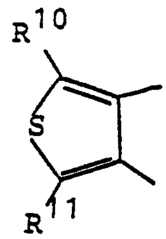

| $R^1$ | $R^2$ | $R^3$ | $R^4$ | $R^5$ | $R^6$ | $R^{10}$ | $R^{11}$ |
|---|---|---|---|---|---|---|---|
| Et | H | H | H | OMe | Me | OMe | OMe |
| Et | H | H | H | OMe | Cl | OMe | OMe |
| Et | H | Cl | H | OEt | H | OMe | OMe |
| Et | H | Br | H | OMe | H | OMe | OMe |
| Et | H | Me | H | OMe | H | OMe | OMe |
| Et | H | Me | H | H | H | OMe | OMe |
| Et | H | H | Me | H | H | OMe | OMe |
| Et | H | H | H | Me | H | OMe | OMe |
| Et | H | Et | H | H | H | OMe | OMe |
| Et | H | H | Et | H | H | OMe | OMe |
| Et | H | H | H | Et | H | OMe | OMe |
| Et | H | H | H | H | Me | OMe | OMe |
| Et | H | Cl | H | H | H | OMe | OMe |
| Et | H | H | Cl | H | H | OMe | OMe |
| Et | H | H | H | Cl | H | OMe | OMe |
| Et | H | H | H | H | Cl | OMe | OMe |
| Et | H | Br | H | H | H | OMe | OMe |
| Et | H | H | Br | H | H | OMe | OMe |
| Et | H | H | H | Br | H | OMe | OMe |
| Et | H | OMe | H | H | H | OMe | OMe |
| Et | H | H | H | OMe | H | OMe | OMe |
| Et | H | H | H | OEt | H | OMe | OMe |
| Et | H | F | H | H | H | OMe | OMe |
| Et | H | H | F | H | H | OMe | OMe |
| Et | H | H | H | F | H | OMe | OMe |
| Et | H | H | H | H | F | OMe | OMe |
| Et | H | H | COOEt | H | H | OMe | OMe |
| Et | H | H | H | COOEt | H | OMe | OMe |

$R^7,R^8,R^9$ = H
T = S

A = 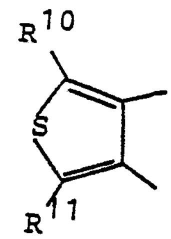

| $R^1$ | $R^2$ | $R^3$ | $R^4$ | $R^5$ | $R^6$ | $R^{10}$ | $R^{11}$ |
|---|---|---|---|---|---|---|---|
| Me | H | H | OMe | H | H | OMe | OMe |
| H | H | H | H | OMe | H | OMe | OMe |
| Me | H | H | H | OMe | H | OMe | OMe |
| Me | H | H | H | OEt | H | OMe | OMe |
| Me | H | F | H | H | H | OMe | OMe |
| ME | H | H | F | H | H | OMe | OMe |
| Me | H | H | H | F | H | OMe | OMe |
| Me | H | H | H | H | F | OMe | OMe |
| Me | H | Me | H | Me | H | OMe | OMe |
| Me | H | Me | Me | H | H | OMe | OMe |
| Me | H | H | Me | Me | H | OMe | OMe |
| Me | H | Me | H | Cl | H | OMe | OMe |
| Me | H | Cl | Cl | H | H | OMe | OMe |
| H | H | Cl | H | Cl | H | OMe | OMe |
| Me | H | Cl | H | Cl | H | OMe | OMe |
| Et | H | Cl | H | Cl | H | OMe | OMe |
| H | H | Cl | H | OMe | H | OMe | OMe |
| H | H | Cl | G | OMe | H | OMe | OMe |
| H | H | H | Cl | OMe | H | OMe | OMe |
| H | H | H | H | OMe | Cl | OMe | OMe |
| H | H | Cl | H | OEt | H | OMe | OMe |
| H | H | Br | H | OMe | H | OMe | OMe |
| Me | H | H | Me | Me | H | H | H |
| Me | H | H | Me | Me | Me | OMe | OMe |
| Me | H | H | OMe | Me | Me | H | H |
| Me | H | H | OMe | Me | Me | OMe | OME |
| H | H | H | Me | OMe | Me | H | H |
| H | H | H | Me | OMe | Me | OMe | OMe |
| Me | H | H | Me | OMe | Me | H | H |
| Me | H | H | Me | OMe | Me | OMe | OMe |
| Me | H | H | Me | OMe | Me | OEt | H |

$R^7, R^8, R^9$ = H
T = S

A = 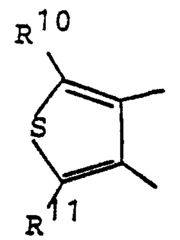

| $R^1$ | $R^2$ | $R^3$ | $R^4$ | $R^5$ | $R^6$ | $R^{10}$ | $R^{11}$ |
|---|---|---|---|---|---|---|---|
| Me | H | H | Cl | OMe | Cl | H | H |
| Me | H | H | Cl | OMe | Cl | OMe | OMe |
| Me | H | H | Me | Cl | Me | H | H |
| Me | H | H | Me | Cl | Me | OMe | OMe |
| Me | H | H | Cl | Me | Cl | H | H |
| H | $-(CH_2)_2-$ | | H | H | H | H | H |
| H | $-(CH_2)_2-$ | | H | H | H | OEt | H |
| H | $-(CH_2)_2-$ | | H | H | H | OMe | OMe |
| H | $-(CH_2)_3-$ | | H | H | H | H | H |
| H | $-(CH_2)_3-$ | | H | H | H | OEt | H |
| H | $-(CH_2)_3-$ | | H | H | H | OMe | OMe |
| H | $-(CH_2)_3-$ | | H | OMe | H | H | H |
| H | $-(CH_2)_3-$ | | H | OMe | H | OMe | OMe |
| $-(CH_2)_4-$ | | H | H | H | H | H | H |
| $-(CH_2)_4-$ | | Me | H | H | H | H | H |
| $-(CH_2)_4-$ | | H | H | Me | H | H | H |
| $-(CH_2)_4-$ | | H | H | OMe | H | H | H |
| H | H | H | $-(CH_2)_4-$ | | H | H | H |
| Me | H | H | $-(CH_2)_4-$ | | H | H | H |
| Me | H | H | $-(CH_2)_4-$ | | H | OMe | OMe |
| H | H | H | H | $-(CH_2)_4-$ | | H | H |
| Me | H | H | H | $-(CH_2)_4-$ | | H | H |
| Me | H | H | H | $-(CH_2)_4-$ | | OMe | OMe |
| All | H | H | H | H | H | H | H |
| All | H | H | H | H | H | OMe | OMe |
| All | H | H | H | Me | H | H | H |
| All | H | H | H | Me | H | OMe | OMe |
| All | H | Me | H | H | H | H | H |
| All | H | Me | H | H | H | OEt | OEt |
| cPent | H | H | H | H | H | H | H |
| cPent | H | H | H | H | H | OEt | OEt |
| cPent | H | H | H | Me | H | H | H |

$R^7, R^8, R^9$ = H
T = S

A = 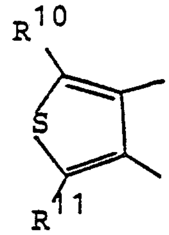

| $R^1$ | $R^2$ | $R^3$ | $R^4$ | $R^5$ | $R^6$ | $R^{10}$ | $R^{11}$ |
|---|---|---|---|---|---|---|---|
| Et | H | Me | H | Me | H | OMe | OMe |
| Et | H | Me | Me | H | H | OMe | OMe |
| Et | H | H | Me | Me | H | OMe | OMe |
| Et | H | Me | H | Cl | H | OMe | OMe |
| Et | H | Cl | Cl | H | H | OMe | OMe |

$R^7, R^8, R^9$ = H
T = SO

A = 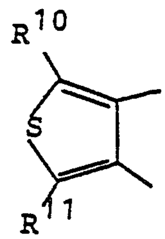

| $R^1$ | $R^2$ | $R^3$ | $R^4$ | $R^5$ | $R^6$ | $R^{10}$ | $R^{11}$ |
|---|---|---|---|---|---|---|---|
| Et | H | Me | H | Me | H | OMe | OMe |
| Et | H | Me | Me | H | H | OMe | OMe |
| Et | H | H | Me | Me | H | OMe | OMe |
| Et | H | Me | H | Cl | H | OMe | OMe |
| Et | H | Cl | Cl | H | H | OMe | OMe |

$R^7, R^8, R^9$ = H

T = SO

A = 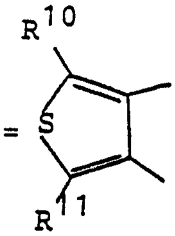

| $R^1$ | $R^2$ | $R^3$ | $R^4$ | $R^5$ | $R^6$ | $R^{10}$ | $R^{11}$ |
|---|---|---|---|---|---|---|---|
| H | H | H | Me | H | H | H | H |
| Me | H | H | Me | H | H | H | H |
| Me | H | H | Me | H | H | OMe | H |
| Me | H | H | Me | H | H | OEt | H |
| Me | H | H | Me | H | H | OMe | OMe |
| Me | H | Me | H | H | H | H | H |
| Me | H | Me | H | H | H | OMe | H |
| Me | H | Me | H | H | H | OEt | H |
| Me | H | H | H | Me | H | H | H |
| Me | H | H | H | Me | H | OMe | H |
| Me | H | H | H | Me | H | OEt | H |
| Me | H | H | H | Me | H | OMe | OMe |
| H | H | H | H | H | Me | H | H |
| Me | H | H | H | H | Me | H | H |
| Me | H | H | H | H | Me | OMe | H |
| H | H | Et | H | H | H | H | H |
| Me | H | Et | H | H | H | H | H |
| Me | H | H | Et | H | H | H | H |
| Me | H | H | H | Et | H | H | H |
| H | H | Cl | H | H | H | H | H |
| Me | H | Cl | H | H | H | H | H |
| Me | H | Cl | H | H | H | OMe | H |
| Me | H | Cl | H | H | H | OEt | H |
| H | H | H | Cl | H | H | H | H |
| Me | H | H | Cl | H | H | H | H |
| Me | H | H | Cl | H | H | OEt | H |
| H | H | H | H | Cl | H | H | H |
| Me | H | H | H | Cl | H | H | H |
| Me | H | H | H | Cl | H | OMe | H |
| H | H | H | H | H | Cl | H | H |
| Me | H | H | H | H | Cl | H | H |

$R^7, R^8, R^9$ = H
T = SO

A = 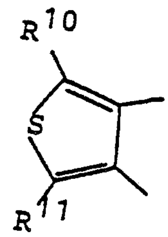

| $R^1$ | $R^2$ | $R^3$ | $R^4$ | $R^5$ | $R^6$ | $R^{10}$ | $R^{11}$ |
|---|---|---|---|---|---|---|---|
| H | H | Br | H | H | H | H | H |
| Me | H | Br | H | H | H | H | H |
| Me | H | Br | H | H | H | OEt | H |
| Me | H | H | Br | H | H | H | H |
| Me | H | H | H | Br | H | H | H |
| Me | H | H | H | Br | H | OEt | H |
| H | H | OMe | H | H | H | H | H |
| Me | H | OMe | H | H | H | H | H |
| Me | H | OMe | H | H | H | OEt | H |
| H | H | H | OMe | H | H | H | H |
| Me | H | H | OMe | H | H | H | H |
| H | H | H | H | OMe | H | H | H |
| Me | H | H | H | OMe | H | H | H |
| Me | H | H | H | OMe | H | OEt | H |
| Me | H | H | H | H | OMe | H | H |
| H | H | OEt | H | H | H | H | H |
| Me | H | OEt | H | H | H | H | H |
| Me | H | OEt | H | H | H | OEt | H |
| Me | H | H | OEt | H | H | H | H |
| Me | H | H | H | OEt | H | H | H |
| H | H | H | H | OEt | H | H | H |
| Me | H | H | H | OEt | H | OEt | H |
| Me | H | F | H | H | H | H | H |
| Me | H | H | F | H | H | H | H |
| Me | H | H | H | F | H | H | H |
| Me | H | H | H | H | F | H | H |

$R^7, R^8, R^9$ = H
T = SO

A = 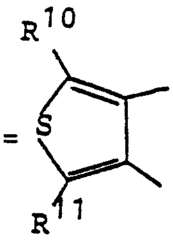

| $R^1$ | $R^2$ | $R^3$ | $R^4$ | $R^5$ | $R^6$ | $R^{10}$ | $R^{11}$ |
|---|---|---|---|---|---|---|---|
| H | H | Me | H | Me | H | H | H |
| Me | H | Me | H | Me | H | H | H |
| Me | H | Me | H | Me | H | OEt | H |
| Me | H | Me | Me | H | H | H | H |
| Me | H | Me | H | H | Me | H | H |
| Me | H | H | Me | Me | H | H | H |
| H | H | H | H | Me | Me | H | H |
| Me | H | H | H | Me | Me | H | H |
| H | H | Me | H | Cl | H | H | H |
| Me | H | Me | H | Cl | H | H | H |
| Me | H | Me | H | Cl | H | OEt | H |
| H | H | Cl | Cl | H | H | H | H |
| Me | H | Cl | Cl | H | H | H | H |
| H | H | Cl | H | Cl | H | H | H |
| Me | H | Cl | H | Cl | H | H | H |
| Me | H | Cl | H | Cl | H | OEt | H |
| Me | H | Cl | H | H | Cl | H | H |
| H | H | H | Cl | Cl | H | H | H |
| Me | H | H | Cl | Cl | H | H | H |
| Me | H | H | H | Cl | Cl | H | H |
| H | H | H | H | Cl | Cl | H | H |
| H | H | Br | H | Br | H | H | H |
| Me | H | Br | H | Br | H | H | H |
| H | H | Cl | H | OMe | H | H | H |
| Me | H | Cl | H | OMe | H | H | H |
| Me | H | Cl | H | OMe | H | OEt | H |
| H | H | OMe | H | Cl | H | H | H |
| Me | H | OMe | H | Cl | H | H | H |
| H | H | H | Cl | OMe | H | H | H |
| Me | H | H | Cl | OMe | H | H | H |
| H | H | H | H | OMe | Cl | H | H |
| Me | H | H | H | OMe | Cl | H | H |

$R^7, R^8, R^9$ = H

T = SO

A = 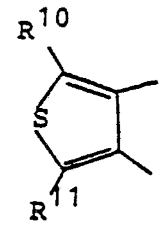

| $R^1$ | $R^2$ | $R^3$ | $R^4$ | $R^5$ | $R^6$ | $R^{10}$ | $R^{11}$ |
|---|---|---|---|---|---|---|---|
| Me | H | H | H | OMe | Cl | OEt | H |
| H | H | Cl | H | OEt | H | H | H |
| Me | H | Cl | H | OEt | H | H | H |
| H | H | Br | H | OMe | H | H | H |
| Me | H | Br | H | OMe | H | H | H |
| H | H | Me | H | OMe | H | H | H |
| Me | H | Me | H | OMe | H | H | H |
| H | H | H | Me | OMe | H | H | H |
| Me | H | H | Me | OMe | H | H | H |
| H | H | H | H | OMe | Me | H | H |
| Me | H | H | H | OMe | Me | H | H |
| Me | H | H | H | OMe | Me | OEt | H |
| Me | H | H | H | OMe | Me | OMe | OMe |
| Me | H | H | H | OMe | Cl | OMe | OMe |
| Me | H | Cl | H | OEt | H | OMe | OMe |
| Me | H | Br | H | OMe | H | OMe | OMe |
| Me | H | Me | H | OMe | H | OMe | OMe |
| Me | H | Me | H | H | H | OMe | OMe |
| Me | H | H | Me | H | H | OMe | OMe |
| H | H | H | H | Me | | OMe | OMe |
| Me | H | H | H | Me | H | OMe | OMe |
| Me | H | Et | H | H | H | OMe | OMe |
| Me | H | H | Et | H | H | OMe | OMe |
| Me | H | H | H | Et | H | OMe | OMe |
| Me | H | H | H | H | Me | OMe | OMe |
| H | H | Cl | H | H | H | OMe | OMe |
| Me | H | Cl | H | H | H | OMe | OMe |
| Me | H | H | Cl | H | H | OMe | OMe |
| H | H | H | H | Cl | H | OMe | OMe |
| Me | H | H | H | Cl | H | OMe | OMe |
| Me | H | H | H | H | Cl | OMe | OMe |
| Me | H | Br | H | H | H | OMe | OMe |
| Me | H | H | Br | H | H | OMe | OMe |
| Me | H | H | H | Br | H | OMe | OMe |
| Me | H | OMe | H | H | H | OMe | OMe |

$R^7,R^8,R^9$ = H
T = SO

A = 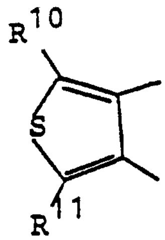

| $R^1$ | $R^2$ | $R^3$ | $R^4$ | $R^5$ | $R^6$ | $R^{10}$ | $R^{11}$ |
|---|---|---|---|---|---|---|---|
| Et | H | H | H | OMe | Me | OMe | OMe |
| Et | H | H | H | OMe | Cl | OMe | OMe |
| Et | H | Cl | H | OEt | H | OMe | OMe |
| Et | H | Br | H | OMe | H | OMe | OMe |
| Et | H | Me | H | OMe | H | OMe | OMe |
| Et | H | Me | H | H | H | OMe | OMe |
| Et | H | H | Me | H | H | OMe | OMe |
| Et | H | H | H | Me | H | OMe | OMe |
| Et | H | Et | H | H | H | OMe | OMe |
| Et | H | H | Et | H | H | OMe | OMe |
| Et | H | H | H | Et | H | OMe | OMe |
| Et | H | H | H | H | Me | OMe | OMe |
| Et | H | Cl | H | H | H | OMe | OMe |
| Et | H | H | Cl | H | H | OMe | OMe |
| Et | H | H | H | Cl | H | OMe | OMe |
| Et | H | H | H | H | Cl | OMe | OMe |
| Et | H | Br | H | H | H | OMe | OMe |
| Et | H | H | Br | H | H | OMe | OMe |
| Et | H | H | H | Br | H | OMe | OMe |
| Et | H | OMe | H | H | H | OMe | OMe |
| Et | H | H | OMe | H | H | OMe | OMe |
| Et | H | H | H | OMe | H | OMe | OMe |
| Et | H | H | H | OEt | H | OMe | OMe |
| Et | H | F | H | H | H | OMe | OMe |
| Et | H | H | F | H | H | OMe | OMe |
| Et | H | H | H | F | H | OMe | OMe |
| Et | H | H | H | H | F | OMe | OMe |

$R^7, R^8, R^9$ = H
T = SO

A = 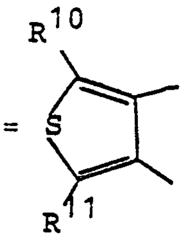

| $R^1$ | $R^2$ | $R^3$ | $R^4$ | $R^5$ | $R^6$ | $R^{10}$ | $R^{11}$ |
|---|---|---|---|---|---|---|---|
| Me | H | H | OMe | H | H | OMe | OMe |
| H | H | H | H | OMe | H | OMe | OMe |
| Me | H | H | H | OMe | H | OMe | OMe |
| Me | H | H | H | OEt | H | OMe | OMe |
| Me | H | F | H | H | H | OMe | OMe |
| ME | H | H | F | H | H | OMe | OMe |
| Me | H | H | H | F | H | OMe | OMe |
| Me | H | H | H | H | F | OMe | OMe |
| Me | H | Me | H | Me | H | OMe | OMe |
| Me | H | Me | Me | H | H | OMe | OMe |
| Me | H | H | Me | Me | H | OMe | OMe |
| Me | H | Me | H | Cl | H | OMe | OMe |
| Me | H | Cl | Cl | H | H | OMe | OMe |
| H | H | Cl | H | Cl | H | OMe | OMe |
| Me | H | Cl | H | Cl | H | OMe | OMe |
| Et | H | Cl | H | Cl | H | OMe | OMe |
| H | H | Cl | H | OMe | H | OMe | OMe |
| H | H | Cl | G | OMe | H | OMe | OMe |
| H | H | H | Cl | OMe | H | OMe | OMe |
| H | H | H | H | OMe | Cl | OMe | OMe |
| H | H | Cl | H | OEt | H | OMe | OMe |
| H | H | Br | H | OMe | H | OMe | OMe |
| Me | H | H | Me | Me | H | H | H |
| Me | H | H | Me | Me | Me | OMe | OMe |
| Me | H | H | OMe | Me | Me | H | H |
| Me | H | H | OMe | Me | Me | OMe | OME |
| H | H | H | Me | OMe | Me | H | H |
| H | H | H | Me | OMe | Me | OMe | OMe |
| Me | H | H | Me | OMe | Me | H | H |
| Me | H | H | Me | OMe | Me | OMe | OMe |
| Me | H | H | Me | OMe | Me | OEt | H |

$R^7, R^8, R^9$ = H

T = SO

A = 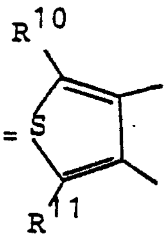

| $R^1$ | $R^2$ | $R^3$ | $R^4$ | $R^5$ | $R^6$ | $R^{10}$ | $R^{11}$ |
|---|---|---|---|---|---|---|---|
| Me | H | H | Cl | OMe | Cl | H | H |
| Me | H | H | Cl | OMe | Cl | OMe | OMe |
| Me | H | H | Me | Cl | Me | H | H |
| Me | H | H | Me | Cl | Me | OMe | OMe |
| Me | H | H | Cl | Me | Cl | H | H |
| H | $-(CH_2)_2-$ | | H | H | H | H | H |
| H | $-(CH_2)_2-$ | | H | H | H | OEt | H |
| H | $-(CH_2)_2-$ | | H | H | H | OMe | OMe |
| H | $-(CH_2)_3-$ | | H | H | H | H | H |
| H | $-(CH_2)_3-$ | | H | H | H | OEt | H |
| H | $-(CH_2)_3-$ | | H | H | H | OMe | OMe |
| H | $-(CH_2)_3-$ | | H | OMe | H | H | H |
| H | $-(CH_2)_3-$ | | H | OMe | H | OMe | OMe |
| $-(CH_2)_4-$ | | H | H | H | H | H | H |
| $-(CH_2)_4-$ | | Me | H | H | H | H | H |
| $-(CH_2)_4-$ | | H | H | Me | H | H | H |
| $-(CH_2)_4-$ | | H | H | OMe | H | H | H |
| H | H | H | $-(CH_2)_4-$ | | H | H | H |
| Me | H | H | $-(CH_2)_4-$ | | H | H | H |
| Me | H | H | $-(CH_2)_4-$ | | H | OMe | OMe |
| H | H | H | H | $-(CH_2)_4-$ | | H | H |
| Me | H | H | H | $-(CH_2)_4-$ | | H | H |
| Me | H | H | H | $-(CH_2)_4-$ | | OMe | OMe |
| All | H | H | H | H | H | H | H |
| All | H | H | H | H | H | OMe | OMe |
| All | H | H | H | Me | H | H | H |
| All | H | H | H | Me | H | OMe | OMe |
| All | H | Me | H | H | H | H | H |
| All | H | Me | H | H | H | OEt | OEt |
| cPent | H | H | H | H | H | H | H |
| cPent | H | H | H | H | H | OEt | OEt |
| cPent | H | H | H | Me | H | H | H |

Abkürzungen:
Me Methyl
Et Ethyl
All Allyl
cPent Cyclopentyl

Die neuen Verbindungen der Formel I und ihre Salze besitzen wertvolle pharmakologische Eigenschaften.

Sie hemmen deutlich die Magensäuresekretion und weisen darüber hinaus eine ausgezeichnete Magen- und Darmschutzwirkung auf.

Unter "Magen-und Darmschutz" wird in diesem Zusammenhang die Verhütung und Behandlung gastrointestinaler Krankheiten, insbesondere gastrointestinaler entzündlicher Krankheiten und Läsionen (wie z.B. Ulcus ventriculi, Ulcus duodeni, Gastritis, hyperazider oder medikamentös bedingter Reizmagen) verstanden, die beispielsweise durch Mikroorganismen, Bakterientoxine, Medikamente (z.B. Antiphlogistika und Antirheumatika), Chemikalien (z.B. Ethanol), Magensäure oder Streßsituationen verursacht werden können.

Aufgrund ihrer ausgezeichneten Eigenschaften sind die substituierten Thienoimidazole der Formel I und ihre pharmakologisch verträglichen Salze für den Einsatz in der Human-und Veterinärmedizin hervorragend geeignet, wobei sie insbesondere zur Behandlung und Prophylaxe von Krankheiten des Magens und Darms und solcher Krankheiten, die auf einer überhöhten Magensäuresekretion beruhen, verwendet werden.

Die Erfindung betrifft daher weiter die erfindungsgemäßen Verbindungen der Formel I zur Anwendung bei der Behandlung und Prophylaxe der vorstehend genannten Krankheiten.

Ebenso umfaßt die Erfindung die Verwendung der erfindungsgemäßen Verbindungen bei der Herstellung von Arzneimitteln, die zur Behandlung und Prophylaxe der vorstehend genannten Krankheiten eingesetzt werden.

Ein weiterer Gegenstand der Erfindung sind Arzneimittel, die ein oder mehrere Verbindungen der allgemeinen Formel I und/oder ihre pharmakologisch verträglichen Salze enthalten.

Die Arzneimittel werden nach an sich bekannten, dem Fachmann geläufigen Verfahren hergestellt. Als Arzneimittel werden die erfindungsgemäßen pharmakologisch wirksamen Verbindungen (= Wirkstoffe) entweder als solche, oder vorzugsweise in Kombination mit geeigneten pharmazeutischen Hilfsstoffen in Form von Tabletten, Dragees, Kapseln, Suppositorien, Emulsionen, Suspensionen oder Lösungen eingesetzt, wobei der Wirkstoffgehalt vorteilhafterweise zwischen 0,1 und 96 Gew.-% beträgt.

Welche Hilfsstoffe für die gewünschte Arzneimittelformulierungen geeignet sind, ist dem Fachmann aufgrund seines Wissens geläufig. Neben Lösemitteln, Gelbildern, Suppositoriengrundlagen, Tabletten-Hilfsstoffen und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Die Wirkstoffe können oral oder parenteral appliziert werden, wobei die orale Applikation bevorzugt ist.

Im allgemeinen hat es sich in der Humanmedizin als vorteilhaft erwiesen, den oder die Wirkstoffe bei oraler Gabe in einer Tagesdosis von etwa 0,01 bis etwa 20 mg/kg Körpergewicht, gegebenenfalls in Form mehrerer, vorzugsweise 1 bis 4 Einzelgaben zur Erzielung des gewünschten Ergebnisses zu verabreichen. Bei der parenteralen Applikation können ähnliche bzw. (insbesondere bei der intravenösen Verabreichung der Wirkstoffe) in der Regel niedrigere Dosierungen zur Anwendung kommen. Die Festlegung der jeweils erforderlichen optimalen Dosierung und Applikationsart der Wirkstoffe kann durch jeden Fachmann aufgrund seines Fachwissens leicht erfolgen.

Sollen die erfindungsgemäßen Verbindungen und/oder ihre Salze zur Behandlung der obengenannten Krankheiten eingesetzt werden, so können die pharmazeutischen Zubereitungen auch einen oder mehrere pharmakologisch aktive Bestandteile anderer Arzneimittelgruppen, wie Antacida, beispielsweise Aluminiumhydroxid, Magnesiumaluminat; Tranquilizer, wie Benzodiazepine, beispielsweise Diazepam; Spasmolytika, wie z.B. Bietamiverin, Camylofin; Anticholinergica, wie z.B. Oxyphencyclimin, Phencarbamid; Lokalanaesthetika, wie z.B. Tetracain, Procain, gegebenenfalls auch Fermente, Vitamine oder Aminosäuren enthalten.

Für eine orale Anwendungsform werden die aktiven Verbindungen mit den dafür üblichen Zusatzstoffen wie Trägerstoffen, Stabilisatoren oder inerten Verdünnungsmitteln vermischt und durch übliche Methoden in geeignete Darreichungsformen gebracht, wie Tabletten, Dragees, Steckkapseln, wäßrige, alkoholische oder ölige Suspensionen oder wäßrige, alkoholische oder ölige Lösungen. Als inerte Träger können z.B. Gummi arabicum, Magnesia, Magnesiumcarbonat, Milchzucker, Glucose oder Stärke, insbesondere Maisstärke verwendet werden. Dabei kann die Zubereitung sowohl als Trocken-oder Feuchtgranulat erfolgen. Als ölige Trägerstoffe oder Lösungsmittel kommen beispielsweise pflanzliche und tierische Öle in Betracht, wie Sonnenblumenöl oder Lebertran.

Zur subkutanen oder intravenösen Applikation werden die aktiven Verbindungen oder deren physiologisch verträgliche Salze, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel für die neuen aktiven Verbindungen und die entsprechenden physiologisch verträglichen Salze kommen z.B. in Frage: Wasser, physiologische Kochsalzlösungen oder Alkohole, z.B. Ethanol, Propanol oder Glycerin, daneben auch Zuckerlösungen wie Glucose-oder Mannitlösungen, oder auch eine Mischung aus den verschiedenen genannten Lösungsmitteln.

Die nachfolgend aufgeführten Beispiele dienen zur Erläuterung der Erfindung, ohne diese jedoch einzuschränken. Die angegebenen Schmelz-und Zersetzungspunkte sind nicht korrigiert oder standardisiert.

Beispiel 1

2-(2-Dimethylaminobenzylmercapto)-1H-thieno[3,4-d]imidazol

Zu einer Lösung von 1,56 g 2-Mercapto-thieno[3,4-d]imidazol in 20 ml wasserfreiem Dimethylacetamid werden 2,9 g 2-Dimethylaminobenzylbromid-Hydrobromid gegeben. Unter schwach exothermer Reaktion - scheidet sich ein kristalliner Niederschlag ab. Man rührt eine Stunde bei Raumtemperatur, versetzt mit Aceton, filtriert die Kristalle ab und trocknet im Luftstrom. Sodann trägt man den kristallinen Feststoff in wäßrige gesättigte $NaHCO_3$-Lösung ein, erwärmt kurz auf dem Dampfbad und filtriert die Kristalle nach Rühren bei Raumtemperatur ab. Nach dem Behandeln mit Aktivkohle in Ethanol und Einengen wird die Lösung mit wenig Aceton zur Kristallisation gebracht.
Kristalline Substanz, Fp. 124-131 °C.

Beispiel 2

2-(2-Dimethylaminobenzylsulfinyl)-1H-thieno[3,4-d]imidazol

Zu einer Lösung von 0,28 g 2-(2-Dimethylaminobenzylmercapto)-1H-thieno[3,4-d]imidazol in 50 ml Methylenchlorid werden nacheinander 50 ml gesättigte Natriumbicarbonatlösung und nach Abkühlung auf 0 bis 5 °C unter Rühren eine Lösung von 0.2 g 3-Chlorperbenzoesäure (85 %ig) in Methylenchlorid gegeben. Nach Abtrennung und Abdestillieren der organischen Phase wird der Rückstand an Kieselgel (Laufmittel Essigester/Methanol = 1:1) chromatographiert.
Kristalle, Fp. 120 °C (Zers.).

Beispiel 3

2-(2-Diethylaminobenzylmercapto)-1H-thieno[3,4-d]imidazol

1,47 g 2-Mercapto-thieno[3,4,-d]imidazol werden mit 3,05 g 2-Diethylaminobenzylbromid-Hydrobromid analog der in Beispiel 1 beschriebenen Vorschrift umgesetzt und aufgearbeitet.
Kristalliner Feststoff, Fp. 124 °C (Zers.).

Beispiel 4

2-(2-Diethylaminobenzylsulfinyl)-1H-thieno[3,4-d]imidazol

1,6 g 2-(2-Diethylaminobenzylmercapto)-1H-thieno[3,4-d]imidazol werden analog der in Beispiel 2 angegebenen Vorschrift umgesetzt und aufgearbeitet.
Kristalliner Feststoff, Fp. 155 °C (Zers.).

Beispiel 5

2-(2-Ethylaminobenzylmercapto)-1H-thieno[3,4-d]imidazol

Zu einer Lösung von 0,60 g 2-Mercapto-thieno[3,4-d]imidazol in 100 ml Aceton gibt man 1,12 g 2-Ethylaminobenzylbromid-Hydrobromid. Nachdem 24 Stunden bei Raumtemperatur gerührt wurde, destilliert man das Lösungsmittel im Vakuum ab und wäscht den kristallinen Rückstand mit Aceton. Anschließend wird das Produkt mit 1n-$NH_4OH$-Lösung versetzt, 2 Stunden gerührt, abgesaugt und mit Wasser gewaschen. Der an der Luft getrocknete Rückstand wird in Methylenchlorid gelöst, mit Aktivkohle versetzt und filtriert. Nach dem Einengen im Vakuum erhält man das farblose, kristalline Produkt.
Fp. 230 °C (Zers.)

Beispiel 6

2-(2-Aminobenzylsulfinyl)-1H-thieno[3,4-d]imidazol-Natriumsalz

Zu 1,50 g 2-Aminobenzylalkohol in 30 ml Tetrahydrofuran läßt man bei -15 °C unter Rühren 1,3 ml Thionylchlorid zutropfen und anschließend 20 Minuten weiterrühren. Diese Lösung wird danach unter Eiskühlung langsam zu einer Lösung von 1,90 g 2-Mercapto-thieno[3,4-d]imidazol in 30 ml 2n-NaOH und 50 ml Ethanol gegeben. Bei Raumtemperatur wird die Lösung eingeengt, mit Wasser versetzt, viermal mit Methylenchlorid extrahiert, die organische Phase über $MgSO_4$ getrocknet und anschließend das Lösungsmittel im Vakuum abdestilliert.
Die erhaltene Mercaptoverbindung (1,5 g) wird in 30 ml Methylenchlorid gelöst und mit 30 ml gesättigter wäßriger $NaHCO_3$-Lösung versetzt. Bei 0 bis -5 °C werden 0,60 g m-Chlorperbenzoesäure - gelöst in Methylenchlorid -hinzugetropft und 20 Minuten nachgerührt. Anschließend wird ein braunes Harz abgetrennt und weitere 0,60 g m-Chlorperbenzoesäure hinzugefügt.
Das ausgefallene Produkt wird abgesaugt und im Luftstrom getrocknet.
Fp. >270 °C

## Ansprüche

1. Verbindung der Formel I

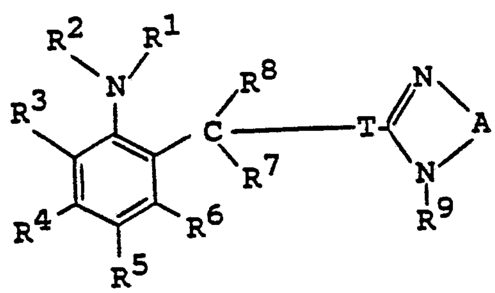

(I)

in welcher
A für

a) 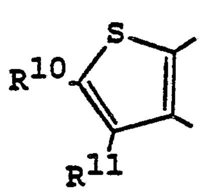 , b) 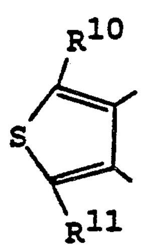 oder c) 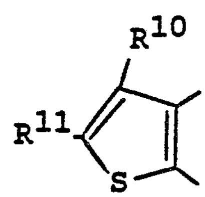

steht,
T -S-, -SO-oder -$SO_2$-bedeutet
$R^1$ und $R^2$ gleich oder verschieden sind und Wasserstoff, ($C_1$-$C_6$)-Alkyl, ($C_3$-$C_6$)-Cycloalkyl, ($C_3$-$C_6$)-Alkenyl

oder ($C_3$-$C_6$)-Alkinyl bedeuten, oder
$R^1$ und $R^2$ gemeinsam für eine Methylenkette -$[CH_2]_n$-stehen, die eine Doppelbindung enthalten kann, worin n = 2,3,4,5 oder 6 ist und worin eine Methylengruppe durch Sauerstoff, Schwefel oder $NR^{12}$ ersetzt sein kann,
$R^3$, $R^4$, $R^5$ und $R^6$ gleich oder verschieden sind und für Wasserstoff, Halogen, Cyano, Trifluormethyl, Benzyloxy, ($C_1$-$C_6$)-Alkyl-Y oder Phenyl-Y-, worin Y Sauerstoff, Schwefel, Sulfinyl, Sulfonyl oder -$[CH_2]_m$-mit m = 0, 1 oder 2 bedeutet, -CO-$R^{13}$, -$SO_2NR^{14}R^{15}$, -O-$COR^{14}$, -$NR^{14}$-$COR^{15}$, -$NR^{14}$-$SO_2R^{15}$ oder -$NR^{14}R^{15}$ stehen,
$R^7$ und $R^8$ gleich oder verschieden sind und Wasserstoff oder Methyl bedeuten,
$R^9$ Wasserstoff, ($C_1$-$C_6$)-Alkanoyl, ($C_1$-$C_6$)-Alkylcarbamoyl oder eine andere physiologisch verträgliche, säurelabile und/oder unter physiologischen Bedingungen abspaltbare $N^{im}$-Schutzgruppe bedeutet,
$R^{10}$ und $R^{11}$ gleich oder verschieden sind und Wasserstoff, Halogen, Cyano, Nitro, Trifluormethyl, ($C_1$-$C_6$)-Alkyl, ($C_1$-$C_6$)-Alkoxy, ($C_1$-$C_6$)-Alkylmercapto, ($C_1$-$C_6$)-Alkylcarbonyl, ($C_1$-$C_6$)-Alkoxycarbonyl, ($C_1$-$C_6$)-Alkylcarbonyloxy, ($C_3$-$C_8$)-Cycloalkyl, Phenyl, Benzyl, Benzyloxy, Phenoxy, Phenylmercapto, Phenylsulfinyl, Phenylsulfonyl, Sulfamoyl, N-($C_1$-$C_4$)-Alkylsulfamoyl oder N,N-Di-($C_1$-$C_4$)-alkylsulfamoyl bedeuten, oder, falls A wie oben unter (a) oder (c) definiert ist, auch gemeinsam -$[CH_2]_n$-mit n = 3, 4, 5 oder 6 bedeuten können, wobei eine oder zwei nicht benachbarte $CH_2$-Gruppen gegebenenfalls durch Sauerstoff ersetzt sind,
$R^{12}$ Wasserstoff, ($C_1$-$C_4$)-Alkyl oder Acyl bedeutet,
$R^{13}$ ($C_1$-$C_5$)-Alkyl, ($C_5$ oder $C_6$)-Cycloalkyl, Hydroxy, ($C_1$-$C_4$)-Alkoxy oder -$NR^{14}R^{15}$ bedeuten,
$R^{14}$ und $R^{15}$ gleich oder verschieden sind und Wasserstoff, ($C_1$-$C_4$)-Alkyl oder Phenyl, das mit ($C_1$-$C_3$)-Alkyl, ($C_1$-$C_4$)-Alkoxy, Trifluormethyl und/oder Halogen, mono-, di-oder trisubstituiert sein kann,
bedeuten, oder
$R^{14}$ und $R^{15}$ gemeinsam für eine Methylenkette -$[CH_2]_q$-stehen,
worin q = 3, 4, 5 oder 6 ist und eine Methylengruppe durch Sauerstoff ersetzt sein kann,
sowie deren physiologisch verträgliche Salze.

2. Verbindung der Formel I gemäß Anspruch 1, in welcher A wie im Anspruch 1 unter (b) definiert ist, sowie deren physiologisch verträgliche Salze..

3. Verbindung der Formel I gemäß einem oder mehreren der Ansprüche 1 und 2, in welcher T für -SO- steht, sowie deren physiologisch verträgliche Salze.

4. Verbindung der Formel I gemäß einem oder mehreren der Ansprüche 1 bis 3, in welcher
$R^1$ und $R^2$ gleich oder verschieden sind und Wasserstoff oder ($C_1$-$C_4$)-Alkyl bedeuten oder gemeinsam eine für Methylenkette -$[CH_2]_n$-mit n = 4 oder 5 stehen, worin eine Methylengruppe durch Sauerstoff ersetzt sein kann,
$R^3$, $R^4$, $R^5$ und $R^6$ gleich oder verschieden sind und Wasserstoff, ($C_1$-$C_4$)-Alkyl oder ($C_1$-$C_4$)-Alkoxy bedeuten,
$R^7$ und $R^8$ für Wasserstoff stehen und
$R^{10}$ und $R^{11}$ gleich oder verschieden sind und Wasserstoff, Methyl oder Methoxy bedeuten,
sowie deren physiologisch verträgliche Salze.

5. Verbindung der Formel I gemäß oder mehreren der Ansprüche 1 bis 4, in welcher
$R^9$ Wasserstoff oder eine physiologisch verträgliche, säurelabile und/oder unter physiologischen Bedingungen abspaltbare $N^{im}$-Schutzgruppe, bedeutet, sowie deren physiologisch verträgliche Salze.

6. Verbindung der Formel I gemäß einem oder mehreren der Ansprüche 1 bis 4, in welcher $R^9$ für ($C_1$-$C_6$)-Alkanoyloxy steht, sowie deren physiologisch verträgliche Salze.

7. Verbindung der Formel I gemäß einem oder mehreren der Ansprüche 1 bis 5, in welcher
A vorzugsweise wie unter (b) definiert ist,
T vorzugsweise eine -SO-Gruppe bedeutet,
$R^1$ und $R^2$ gleich oder verschieden sind und Methyl oder Ethyl bedeuten,
$R^3$, $R^4$, $R^5$ und $R^6$ für Wasserstoff oder 1 oder 2 dieser Reste für ($C_1$-$C_4$)-Alkyl steht/stehen,
$R^7$ und $R^8$ jeweils für Wasserstoff stehen,
$R^9$ Wasserstoff oder ($C_1$-$C_6$)-Alkanoyloxy bedeutet und
$R^{10}$ und $R^{11}$ gleich oder verschieden sind und Methyl oder Methoxy bedeuten,
sowie deren physiologisch verträgliche Salze.

8. 2-(2-Dimethylaminobenzylsulfinyl)-1H-thieno[3,4-d]-imidazol und dessen physiologisch verträgliche Salze.

9. 2-(2-Diethylaminobenzylsulfinyl)-1H-thieno[3,4-d]-imidazol und dessen physiologisch verträgliche Salze.

10. Verfahren zur Herstellung einer Verbindung der Formel I gemäß einem oder mehreren der Ansprüche 1 bis 9, dadurch gekennzeichnet, daß man eine Verbindung der Formel II

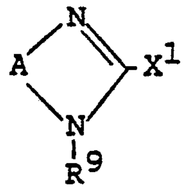

(II)

in welcher
A und $R^9$ wie in Anspruch 1 definiert sind und
$X^1$ i. eine Abgangsgruppe oder
ii. -SH oder -S bedeutet
umsetzt mit einer Verbindung der Formel III

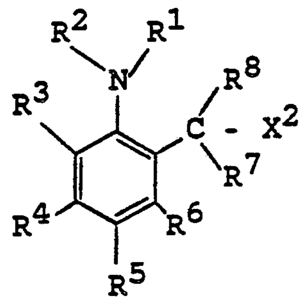

(III)

in welcher
$R^1$, $R^2$, $R^3$, $R^4$, $R^5$, $R^6$, $R^7$ und $R^8$ wie in Anspruch 1 definiert sind
und
$X^2$ im obengenannten Fall i. -SH oder -S und
im obengenannten Fall ii. eine Abgangsgruppe bedeutet,
sowie
i. in Verbindungen der Formel I (eine) gegebenenfalls vorhandene -S-Gruppe gewünschtenfalls zu der -SO-Gruppe oxidiert,
ii. in Verbindungen der Formel I (eine gegebenenfalls vorhandene -SO-Gruppe gewünschtenfalls zur -S-Gruppe reduziert,
iii. Verbindungen der Formel I, worin $R^9$ für Wasserstoff steht, gewünschtenfalls acyliert,
iv. Verbindungen der Formel I, worin $R^9$ nicht Wasserstoff bedeutet, gewünschtenfalls verseift und
v. Verbindungen der Formel I gewünschtenfalls in ihre physiologisch verträglichen Salze überführt,
wobei zwei oder mehr Maßnahmen i.-iv. auch in einer anderen als der angegebenen Reihenfolge ausgeführt werden können.

11. Verbindung gemäß einem oder mehreren der Ansprüche 1 bis 9 zur Anwendung als Heilmittel.

12. Verbindung gemäß einem oder mehreren der Ansprüche 1 bis 9 zur Anwendung als Magensäuresekretionshemmer.

13. Mittel enthaltend eine Verbindung gemäß einem oder mehreren der Ansprüche 1 bis 9, 11 oder 12.

14. Verfahren zur Herstellung eines Mittels gemäß Anspruch 13, dadurch gekennzeichnet, daß man eine Verbindung gemäß einem oder mehreren der Ansprüche 1 bis 9, 11 oder 12 in eine geeignete Darreichungsform bringt.

Patentansprüche für die folgenden Vertragsstaaten: ES und GR

1. Verfahren zur Herstellung einer Verbindung der Formel I

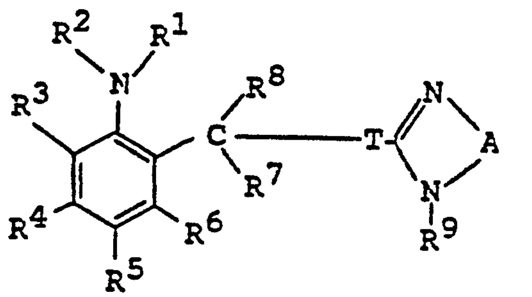

(I)

in welcher
A für

a) 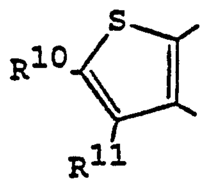 , b) 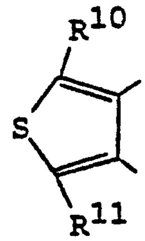 oder c) 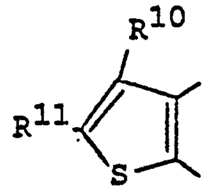

steht,
T -S-, -SO-oder $-SO_2$-bedeutet
$R^1$ und $R^2$ gleich oder verschieden sind und Wasserstoff, $(C_1\text{-}C_6)$-Alkyl, $(C_3\text{-}C_6)$-Cycloalkyl, $(C_3\text{-}C_6)$-Alkenyl oder $(C_3\text{-}C_6)$-Alkinyl bedeuten,
$R^1$ und $R^2$ gemeinsam für eine Methylenkette $-[CH_2]_n$-stehen, die eine Doppelbindung enthalten kann, worin n = 2,3,4,5 oder 6 ist und worin eine Methylengruppe durch Sauerstoff, Schwefel oder $NR^{12}$ ersetzt sein kann,
$R^3$, $R^4$, $R^5$ und $R^6$ gleich oder verschieden sind und für Wasserstoff, Halogen, Cyano, Trifluormethyl, Benzyloxy, $(C_1\text{-}C_6)$-Alkyl-Y oder Phenyl-Y-, worin Y Sauerstoff, Schwefel, Sulfinyl, Sulfonyl oder $-[CH_2]_m$-mit m = 0, 1 oder 2 bedeutet, $-CO\text{-}R^{13}$, $-SO_2NR^{14}R^{15}$, $-O\text{-}COR^{14}$, $-NR^{14}\text{-}COR^{15}$, $-NR^{14}\text{-}SO_2R^{15}$ oder $-NR^{14}R^{15}$ stehen,
$R^7$ und $R^8$ gleich oder verschieden sind und Wasserstoff oder Methyl bedeuten,
$R^9$ Wasserstoff, $(C_1\text{-}C_6)$-Alkanoyl, $(C_1\text{-}C_6)$-Alkylcarbamoyl oder eine andere physiologisch verträgliche, säurelabile und/oder unter physiologischen Bedingungen abspaltbare $N^{im}$-Schutzgruppe bedeutet,
$R^{10}$ und $R^{11}$ gleich oder verschieden sind und Wasserstoff, Halogen, Cyano, Nitro, Trifluormethyl, $(C_1\text{-}C_6)$-Alkyl, $(C_1\text{-}C_6)$-Alkoxy, $(C_1\text{-}C_6)$-Alkylmercapto, $(C_1\text{-}C_6)$-Alkylcarbonyl, $(C_1\text{-}C_6)$-Alkoxycarbonyl, $(C_1\text{-}C_6)$-Alkylcarbonyloxy, $(C_3\text{-}C_8)$-Cycloalkyl, Phenyl, Benzyl, Benzyloxy, Phenoxy, Phenylmercapto, Phenylsulfinyl, Phenylsulfonyl, Sulfamoyl, N-$(C_1\text{-}C_4)$-Alkylsulfamoyl oder N,N-Di-$(C_1\text{-}C_4)$-alkylsulfamoyl bedeuten, oder, falls A wie oben unter (a) oder (c) definiert ist, auch gemeinsam $-[CH_2]_n$-mit n = 3, 4, 5 oder 6 bedeuten können, wobei eine oder zwei nicht benachbarte $CH_2$-Gruppen gegebenenfalls durch Sauerstoff ersetzt sind,
$R^{12}$ Wasserstoff, $(C_1\text{-}C_4)$-Alkyl oder Acyl bedeutet,
$R^{13}$ $(C_1\text{-}C_5)$-Alkyl, ($C_5$ oder $C_6$)-Cycloalkyl, Hydroxy, $(C_1\text{-}C_4)$-Alkoxy oder $-NR^{14}R^{15}$ bedeuten,
$R^{14}$ und $R^{15}$ gleich oder verschieden sind und Wasserstoff, $(C_1\text{-}C_4)$-Alkyl oder Phenyl, das mit $(C_1\text{-}C_3)$-Alkyl, $(C_1\text{-}C_4)$-Alkoxy, Trifluormethyl und/oder Halogen, mono-, di-oder trisubstituiert sein kann, bedeuten, oder
$R^{14}$ und $R^{15}$ gemeinsam für eine Methylenkette $-[CH_2]_q$-stehen,
worin q = 3, 4, 5 oder 6 ist und eine Methylengruppe durch Sauerstoff ersetzt sein kann,
oder deren physiologisch verträglichen Salzes, dadurch gekennzeichnet, daß man eine Verbindung der Formel II

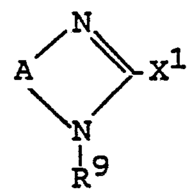

(II)

in welcher
A und $R^9$ wie in Anspruch 1 definiert sind und
$X^1$ i. eine Abgangsgruppe oder
ii. -SH oder -S bedeutet
umsetzt mit einer Verbindung der Formel III

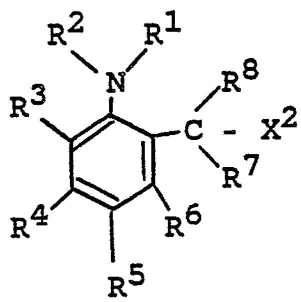

(III)

in welcher
$R^1$, $R^2$, $R^3$, $R^4$, $R^5$, $R^6$, $R^7$ und $R^8$ wie in Anspruch 1 definiert sind
und
$X^2$ im obengenannten Fall i. -SH oder -S und im obengenannten Fall ii. eine Abgangsgruppe bedeutet,
sowie
i. in Verbindungen der Formel I (eine) gegebenenfalls vorhandene -S-Gruppe gewünschtenfalls zu der -SO-Gruppe oxidiert,
ii. in Verbindungen der Formel I (eine) gegebenenfalls vorhandene -SO-Gruppe gewünschtenfalls zur -S-Gruppe reduziert,
iii. Verbindungen der Formel I, worin $R^9$ für Wasserstoff steht, gewünschtenfalls acyliert,
iv. Verbindungen der Formel I, worin $R^9$ nicht Wasserstoff bedeutet, gewünschtenfalls verseift und
v. Verbindungen der Formel I gewünschtenfalls in ihre physiologisch verträglichen Salze überführt,
wobei zwei oder mehr Maßnahmen i.-iv. auch in einer anderen als der angegebenen Reihenfolge ausgeführt werden können.

2. Verfahren gemäß Anspruch 1, dadurch gekennzeichnet, daß man eine Verbindung der Formel I, in welcher A wie im Anspruch 1 unter (b) definiert ist oder deren physiologisch verträgliche Salze herstellt.

3. Verfahren gemäß einem oder mehreren der Ansprüche 1 und 2, dadurch gekennzeichnet, daß man eine Verbindung der Formel I, in welcher T für -SO-steht oder deren physiologisch verträgliche Salze herstellt.

4. Verfahren gemäß einem oder mehreren der Ansprüche 1 bis 3, dadurch gekennzeichnet, daß man eine Verbindung der Formel I, in welcher
$R^1$ und $R^2$ gleich oder verschieden sind und Wasserstoff oder $(C_1\text{-}C_4)$-Alkyl bedeuten oder gemeinsam für eine Methylenkette $-[CH_2]_n$-mit $n = 4$ oder 5 stehen, worin eine Methylengruppe durch Sauerstoff ersetzt sein kann,
$R^3$, $R^4$, $R^5$ und $R^6$ gleich oder verschieden sind und Wasserstoff, $(C_1\text{-}C_4)$-Alkyl oder $(C_1\text{-}C_4)$-Alkoxy bedeuten,
$R^7$ und $R^8$ für Wasserstoff stehen und
$R^{10}$ und $R^{11}$ gleich oder verschieden sind und Wasserstoff, Methyl oder Methoxy bedeuten
oder deren physiologisch verträgliches Salz herstellt.

5. Verfahren gemäß einem oder mehreren der Ansprüche 1 bis 4, dadurch gekennzeichnet, daß man eine Verbindung der Formel I, in welcher
$R^9$ Wasserstoff oder eine physiologisch verträgliche, säurelabile und/oder unter physiologischen Bedingungen abspaltbare $N^{im}$ -Schutzgruppe, bedeutet oder deren physiologisch verträgliches Salz herstellt.

6. Verfahren gemäß einem oder mehreren der Ansprüche 1 bis 4, dadurch gekennzeichnet, daß man eine Verbindung der Formel I, in welcher $R^9$ für
$(C_1\text{-}C_6)$-Alkanoyloxy steht oder deren physiologisch verträgliches Salz herstellt.

7. Verfahren gemäß einem oder mehreren der Ansprüche 1 bis 5, dadurch gekennzeichnet, daß man eine Verbindung der Formel I, in welcher
A vorzugsweise wie unter (b) definiert ist,
T vorzugsweise eine -SO-Gruppe bedeutet,
$R^1$ und $R^2$ gleich oder verschieden sind und Methyl oder Ethyl bedeuten,
$R^3$, $R^4$, $R^5$ und $R^6$ für Wasserstoff oder 1 oder 2 dieser Reste für ($C_1$-$C_4$)-Alkyl steht/stehen
$R^7$ und $R^8$ jeweils für Wasserstoff stehen
$R^9$ Wasserstoff oder ($C_1$-$C_6$)-Alkanoyloxy bedeutet und
$R^{10}$ und $R^{11}$ gleich oder verschieden sind und Methyl oder Methoxy bedeuten
oder deren physiologisch verträgliches Salz herstellt.

8. Verfahren gemäß einem oder mehreren der Ansprüche 1 bis 7 zur Herstellung von 2-(2-Dimethylaminobenzylsulfinyl)-1H-thieno[3,4-d]imidazol oder dessen physiologisch verträglichen Salzes.

9. Verfahren gemäß einem oder mehreren der Ansprüche 1 bis 7 zur Herstellung von 2-(2-Diethylaminobenzylsulfinyl)-1H-thieno[3,4-d]-imidazol oder dessen physiologisch verträglichen Salzes.

10. Verfahren zur Herstellung eines Mittels enthaltend eine Verbindung herstellbar gemäß einem oder mehreren der Ansprüche 1 bis 9, dadurch gekennzeichnet, daß man diese Verbindung in eine geeignete Darreichungsform bringt.